# EUROPEAN PATENT APPLICATION

(11) **EP 4 083 629 A1**
(43) Date of publication of application: **02.11.2022**
(21) Application number: 21170702.1
(22) Date of filing: 27.04.2021
(51) Int. Cl.: G01N 33/68

(54) **NON-INVASIVE DIAGNOSTIC PREDICTION OF TREATMENT SUCCESS IN ALLERGEN SPECIFIC IMMUNOTHERAPY**

(71) Applicant: Klinikum rechts der Isar der Technischen Universität München, 81675 München (DE)
(72) Inventor: ZISSLER, Ulrich, 83376 Truchtlaching (DE); SCHMIDT-Weber, Carsten, 80939 München (DE); SCHUBERT, Benjamin, 81541 München (DE); CHAKER, Adam, 81927 München (DE); JAKWERTH, Constanze, 80997 München (DE)
(74) Representative: Engelhard, Markus

(57) **Abstract**

The present invention relates to a method of predicting and/or monitoring therapeutic success of an allergen-specific immunotherapy (AIT) in a patient. The present invention further relates to a method of determining an allergic response to an allergen in a patient. The present invention also relates to a kit for predicting and/or monitoring therapeutic success of an allergen-specific immunotherapy (AIT) in a patient and/or for determining an allergic response to an allergen in a patient. Furthermore, the present invention relates to a use of a set of biomarkers for predicting and/or monitoring therapeutic success of an allergen-specific immunotherapy and/or for determining an allergic response to an allergen.

## Description

### FIELD OF THE INVENTION

The present invention relates to a method of predicting and/or monitoring therapeutic success of an allergen-specific immunotherapy (AIT) in a patient. The present invention further relates to a method of determining an allergic response to an allergen in a patient. The present invention also relates to a kit for predicting and/or monitoring therapeutic success of an allergen-specific immunotherapy (AIT) in a patient and/or for determining an allergic response to an allergen in a patient. Furthermore, the present invention relates to a use of a set of biomarkers for predicting and/or monitoring therapeutic success of an allergen-specific immunotherapy and/or for determining an allergic response to an allergen.

### BACKGROUND OF THE INVENTION

For patients with allergic diseases such as allergic rhinitis and allergic asthma, there are different approaches for ameliorating the condition. For example, the patients can try to prevent exposure to the respective allergen. However, such approach is severely limited in that such exposure cannot always be avoided without a severe impairment of the quality of life. Furthermore, there is a range of therapeutic treatments available, mainly based on treatment with antihistamines and/or glucocorticoids. However, such a therapy is frequently accompanied with side effects and has a tendency to lose efficacy over time. Moreover, long-term intake of medication may lead to cumulated adverse-effects especially in the case of steroids. Importantly, a symptomatic medication does not change the course of disease.

In contrast to these approaches and as a third possibility, patients, prior to exposure to the seasonally related allergens, may undergo a desensitization or hyposensitization allergen-treatment such that they get artificial exposure to defined doses of allergen and thereby hopefully become less sensitive to it. This latter therapy is also sometimes referred to as allergen-specific immunotherapy (AIT) and involves the artificial and repeated exposure to defined doses of an allergen, for example by subcutaneous application with a syringe or by sublingual administration. Such allergen-specific immunotherapy typically is prolonged over several years and may not be effective or suitable for every patient treated therewith. For this reason, there is a need in the art to be able to determine and predict an individual patient's therapeutic response to an allergen-specific immunotherapy. Particularly, there is a need for means that allow to monitor and/or predict the therapeutic success of an AIT.

Biomarkers of allergenic responses have been studied [1, 2]. However, there is still the need for methods that allow to determine and predict the effectiveness of an allergen-specific immunotherapy towards an individual patient. Being able to predict, at an early stage of such treatment, whether or not it is at all useful to conduct such an allergen-specific immunotherapy would be of great benefit for the respective patient. Accordingly, there is a need for means that allow to monitor and/or predict therapeutic success of an allergen-specific immunotherapy in a patient. Furthermore, there is a need for means that allow to assess the immunological status of a patient, for example whether a patient is allergic to an allergen. Moreover, there is a need for assessing allergenic responses, such as allergenic responses in an allergen-specific immunotherapy, in a non-invasively obtained sample.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of predicting and/or monitoring therapeutic success of an allergen-specific immunotherapy (AIT) in a patient, wherein said method comprises the steps:
a) providing a first sample of said patient obtained at a first time point and a second sample of said patient obtained at a second time point,
b) determining, in each of said samples of said patient, a biomarker level of each biomarker of a set of biomarkers,
   wherein said set of biomarkers comprises at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18,
   wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5,
c) predicting and/or monitoring therapeutic success of an allergen-specific immunotherapy in said patient, wherein a normalization of said biomarker level of one or more biomarkers of said biomarkers selected from said first biomarker collection, in said second sample compared to said first sample, indicates that said immunotherapy is successful.

In one embodiment, said normalization of step c) is an approximation to and/or attainment of a reference value for the respective biomarker level, preferably a reference value of a healthy individual, and/or is an approximation to and/or attainment of a respective biomarker level determined in a reference sample, preferably a reference sample of a healthy individual.

In one embodiment, said set of biomarkers further comprises one or more biomarkers selected from a second biomarker collection consisting of CCL20, CCL23, CSF-1, AREG, TSLP, CD40, IFN-γ, IL-4, IL-13, and IL-5, and said method comprises a step of standardizing said biomarker level of one or more biomarkers of said biomarkers selected from said first biomarker collection using a biomarker level of one or more biomarkers selected from said second biomarker collection.

In one embodiment, said set of biomarkers comprises
at least one anti-inflammatory biomarker selected from SCGB1A1, IL-7, Serpin Gi, IL-37, and NOG, preferably selected from SCGB1A1, Serpin Gi, and IL-37,
at least one pro-inflammatory biomarker selected from CCL-27, IL-24, POSTN, CCL26, CSF-3, and IL-18, preferably selected from CCL-27 and IL-24,
optionally, a standardizing biomarker selected from CCL20, CCL23, CSF-1, AREG, TSLP, CD40, IFN-γ, IL-4, IL-13, and IL-5,
wherein, preferably, said normalization of step c) comprises an increase in said biomarker level of one or more biomarkers of said at least one anti-inflammatory biomarker in said second sample compared to said first sample, and/or comprises a decrease in said biomarker level of one or more biomarkers of said at least one pro-inflammatory biomarker in said second sample compared to said first sample.

In one embodiment, said set of biomarkers comprises biomarkers SCGB1A1, CCL-27, Serpin Gi, IL-37, and IL-24.

In one embodiment, said sample is a sample noninvasively obtained from said patient, preferably a nasal secretion sample. In one embodiment, said first sample is a sample noninvasively obtained from said patient, preferably a nasal secretion sample. In one embodiment, said second sample is a sample noninvasively obtained from said patient, preferably a nasal secretion sample.

In one embodiment, said biomarker levels are determined by determining a protein level and/or a nucleic acid level, preferably a protein level, of each of said biomarkers in said sample; and/or
said biomarker levels are determined using a method selected from high-performance liquid chromatography (HPLC), liquid chromatography-mass spectrometry (LC/MS), enzyme-linked immunosorbent assay (ELISA), protein immunoprecipitation, immunoelectrophoresis, Western blot, protein immunostaining, microarray, PCR such as real time PCR, next generation sequencing, transcriptome-based assays, and capillary test systems.

In one embodiment, said biomarker levels are determined by determining a protein level and/or a nucleic acid level, preferably a protein level, of each of said biomarkers in said first sample and said second sample.

In one embodiment, said biomarker level of each of said biomarkers is determined in said first sample and/or said second sample using a method selected from high-performance liquid chromatography (HPLC), liquid chromatography-mass spectrometry (LC/MS), enzyme-linked immunosorbent assay (ELISA), protein immunoprecipitation, immunoelectrophoresis, Western blot, protein immunostaining, microarray, PCR such as real time PCR, next generation sequencing, transcriptome-based assays, and capillary test systems.

In one embodiment, said allergen-specific immunotherapy comprises
- an initial induction phase, wherein an allergen is repeatedly administered to a patient in increasing doses of said allergen up to a maximum dose effective to induce immunologic tolerance to said allergen in said patient, and
- a maintenance phase, wherein said allergen is administered repeatedly to said patient at said maximum dose,
and, in said method, said biomarker level of each biomarker of said set of biomarkers is determined in the first 1 - 7 weeks of the maintenance phase after the initial induction phase.

In one embodiment, said therapeutic success is measured by a patient-assessed retrospective assessment of seasonal allergic symptoms (RAAS), or by combined symptom medication score (CSMS) or by visual analog scale (VAS).

In one embodiment, said allergen is an allergen involved in any allergic disease selected from allergic rhinitis, bee sting allergy, food allergies, hay fever, and allergic asthma, and/or said allergen is selected from food allergens, pollen, in particular grass pollen, tree pollen and weed pollen, other airborne allergens, such as fungi, fungal spores, dust, mites, and animal dander.

In a further aspect, the present invention relates to a method of determining an allergic response to an allergen in a patient, wherein said method comprises the steps:
i) providing at least one sample of a patient,
   wherein, optionally, said providing at least one sample comprises providing a first sample of said patient obtained at a first time point, preferably said first time point being prior to an allergen exposure, and a second sample of said patient obtained at a second time point, preferably said second time point being after said allergen exposure,
ii) determining, in said at least one sample of said patient, optionally in said first sample and said second sample, a biomarker level of each biomarker of a set of biomarkers,
   wherein said set of biomarkers comprises at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18,
   wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5,
iii) determining an allergic reaction to said allergen in said patient, if said biomarker level of one or more biomarkers of said biomarkers selected from said first biomarker collection in said sample is abnormal,
   wherein, optionally, said biomarker level of one or more biomarkers of said biomarkers selected from said first biomarker collection is abnormal in said second sample compared to said first sample.

In one embodiment, said biomarker level being abnormal comprises said biomarker level deviating from a reference value for the respective biomarker level, preferably a reference value of a healthy individual, and/or
comprises said biomarker level deviating from a respective biomarker level determined in a reference sample, preferably a reference sample of a healthy individual, and/or
comprises said biomarker level determined in said second sample, said second sample obtained after an allergen exposure, deviating from said biomarker level determined in said first sample, said first sample obtained prior to said allergen exposure.

In one embodiment, said set of biomarkers further comprises one or more biomarkers selected from a second biomarker collection consisting of CCL20, CCL23, CSF-1, AREG, TSLP, CD40, IFN-γ, IL-4, IL-13, and IL-5, and said method comprises a step of standardizing said biomarker level of one or more biomarkers of said biomarkers selected from said first biomarker collection using a biomarker level of one or more biomarkers selected from said second biomarker collection.

In one embodiment, said set of biomarkers comprises
at least one anti-inflammatory biomarker selected from SCGB1A1, IL-7, Serpin Gi, IL-37, and NOG, preferably selected from SCGB1A1, Serpin Gi, and IL-37,
at least one pro-inflammatory biomarker selected from CCL-27, IL-24, POSTN, CCL26, CSF-3, and IL-18, preferably selected from CCL-27 and IL-24,
optionally, a standardizing biomarker selected from CCL20, CCL23, CSF-1, AREG, TSLP, CD40, IFN-γ, IL-4, IL-13, and IL-5,
wherein, preferably, said biomarker level being abnormal comprises a decrease in said biomarker level of one or more biomarkers of said at least one anti-inflammatory biomarker in said sample compared to a reference value and/or reference sample, and/or comprises an increase in said biomarker level of one or more biomarkers of said at least one pro-inflammatory biomarker in said sample compared to a reference value and/or reference sample,
wherein, optionally, said reference sample is a first sample obtained prior to an allergen exposure.

In one embodiment, said set of biomarkers comprises biomarkers SCGB1A1, CCL-27, Serpin Gi, IL-37, and IL-24.

In one embodiment, said sample is a sample noninvasively obtained from said patient, preferably a nasal secretion sample. In one embodiment, said first sample is a sample noninvasively obtained from said patient, preferably a nasal secretion sample. In one embodiment, said second sample is a sample noninvasively obtained from said patient, preferably a nasal secretion sample.

In one embodiment, said biomarker levels are determined by determining a protein level and/or a nucleic acid level, preferably a protein level, of each of said biomarkers in said sample; and/or
said biomarker levels are determined using a method selected from high-performance liquid chromatography (HPLC), liquid chromatography-mass spectrometry (LC/MS), enzyme-linked immunosorbent assay (ELISA), protein immunoprecipitation, immunoelectrophoresis, Western blot, protein immunostaining, microarray, PCR such as real time PCR, next generation sequencing, transcriptome-based assays, and capillary test systems.

In one embodiment, said biomarker levels are determined by determining a protein level and/or a nucleic acid level, preferably a protein level, of each of said biomarkers in said first sample and said second sample.

In one embodiment, said biomarker level of each of said biomarkers is determined in said first sample and/or said second sample using a method selected from high-performance liquid chromatography (HPLC), liquid chromatography-mass spectrometry (LC/MS), enzyme-linked immunosorbent assay (ELISA), protein immunoprecipitation, immunoelectrophoresis, Western blot, protein immunostaining, microarray, PCR such as real time PCR, next generation sequencing, transcriptome-based assays, and capillary test systems.

In one embodiment, said allergen is an allergen involved in any allergic disease selected from allergic rhinitis, bee sting allergy, food allergies, hay fever, and allergic asthma, and/or said allergen is selected from food allergens, pollen, in particular grass pollen, tree pollen and weed pollen, other airborne allergens, such as fungi, fungal spores, dust, mites, and animal dander.

In a further aspect, the present invention relates to a kit for predicting and/or monitoring therapeutic success of an allergen-specific immunotherapy (AIT) in a patient and/or for determining an allergic response to an allergen in a patient, comprising:
- means to obtain, preferably noninvasively obtain, a sample from said patient, wherein, optionally, said sample is a nasal secretion of said patient;
- means to perform a quantitative determination of a biomarker level of each biomarker of a set of biomarkers,
   wherein said set of biomarkers comprises at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18,
   wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5,
- optionally, a set of instructions for use of said kit, said instructions indicating a therapeutic success of an allergen-specific immunotherapy (AIT) in said patient, if a biomarker level of one or more biomarkers of said biomarkers selected from said first biomarker collection is normalized in a second sample compared to a first sample; and/or
   a set of instructions for use of said kit, said instructions indicating an allergic response to an allergen in said patient, if a biomarker level of one or more biomarkers of said biomarkers selected from said first biomarker collection in said sample is abnormal, preferably abnormal compared to a reference value and/or reference sample, and/or abnormal in a second sample obtained after an allergen exposure compared to a first sample obtained prior to said allergen exposure.

In one embodiment, said set of biomarkers comprises biomarkers SCGB1A1, CCL-27, Serpin Gi, IL-37, and IL-24.

In this aspect, said predicting and/or monitoring therapeutic success of an allergen-specific immunotherapy (AIT) in a patient, said determining an allergic response to an allergen in a patient, said sample, said biomarker level, said determination of a biomarker level, said set of biomarkers, said sample, said first sample, said second sample, said normalized, said abnormal, said allergen, said reference value, and said reference sample are as defined above.

In a further aspect, the present invention relates to a use of a set of biomarkers for predicting and/or monitoring therapeutic success of an allergen-specific immunotherapy and/or for determining an allergic response to an allergen,
wherein said set of biomarkers comprises at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18,
wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5.

In one embodiment, said set of biomarkers comprises biomarkers SCGB1A1, CCL-27, Serpin Gi, IL-37, and IL-24.

In this aspect, said predicting and/or monitoring therapeutic success of an allergen-specific immunotherapy (AIT) in a patient, said determining an allergic response to an allergen, said allergen, and said set of biomarkers are as defined above.

In a further aspect, the present invention relates to a set of biomarkers,
wherein said set of biomarkers comprises at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18,
wherein, optionally, said set of biomarkers further comprises one or more biomarkers selected from a second biomarker collection consisting of CCL20, CCL23, CSF-1, AREG, TSLP, CD40, IFN-D, IL-4, IL-13, and IL-5.

In one embodiment, said set of biomarkers comprises
at least one anti-inflammatory biomarker selected from SCGB1A1, IL-7, Serpin Gi, IL-37, and NOG, preferably selected from SCGB1A1, Serpin Gi, and IL-37,
at least one pro-inflammatory biomarker selected from CCL-27, IL-24, POSTN, CCL26, CSF-3, and IL-18, preferably selected from CCL-27 and IL-24,
optionally, a standardizing biomarker selected from CCL20, CCL23, CSF-1, AREG, TSLP, CD40, IFN-γ, IL-4, IL-13, and IL-5.

In one embodiment, said set of biomarkers comprises biomarkers SCGB1A1, CCL-27, Serpin Gi, IL-37, and IL-24.

In a further aspect, the present invention relates to a method of allergen-specific immunotherapy, comprising administering, preferably repeatedly administering, an effective amount of an allergen to a patient in need thereof, further comprising predicting and/or monitoring therapeutic success of the allergen-specific immunotherapy using a set of biomarkers, wherein said set of biomarkers comprises at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18, wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5.

In this aspect, said predicting and/or monitoring therapeutic success of the allergen-specific immunotherapy, said administering, and said set of biomarkers are as defined above.

### DETAILED DESCRIPTION

The present invention aims at predicting therapeutic success of an allergen-specific immunotherapy in a patient and/or at determining whether a patient is allergic to an allergen. The present invention further aims at providing means for predicting and/or monitoring therapeutic success of an allergen-specific immunotherapy and/or means for determining an allergic response to an allergen. The present inventors provide a set of biomarkers for use in the methods and use of the invention. The present invention relates to quantification and optionally combinatorial analysis of epithelial biomarkers in the nasal secretion of patients, namely biomarkers of a set of biomarkers comprising a first biomarker collection consisting of SCGB1A1, IL-7, Serpin Gi, IL-37, CSF-3, NOG, CCL-27, IL-24, POSTN, CCL26, and IL-18. The quantification and analysis of epithelial biomarkers in a sample, such as a nasal secretion sample, can be used for monitoring of inflammatory responses, such as type 2 inflammatory responses, for example allergies. Furthermore, the quantification and analysis of epithelial biomarkers in the nasal secretion of the patient can be used to monitor the success of a therapeutic intervention. Type II allergies, which are cytotoxic allergies, typically comprise an antibody-mediated immune reaction against cells of the own body. By determining the biomarker level of each biomarker of a set of biomarkers comprising at least two biomarkers of a first biomarker collection consisting of SCGB1A1, IL-7, Serpin Gi, IL-37, CSF-3, NOG, CCL-27, IL-24, POSTN, CCL26, and IL-18, it can be assessed whether an allergic response is present in a patient and whether an allergen specific immunotherapy is likely to be successful. The inventors have found that a set of biomarkers can be used for analyzing different biological processes, such as acute inflammatory immune reactions in nasal epithelial cells, seasonal allergic reactions, responses to allergen specific immune therapies, as well as successful convalescence, particularly a set of biomarkers comprising at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18.

The present invention further provides a test kit making use of the identified biomarkers and biomarker combinations. The sample, such as nasal secretion sample, can be contacted with the test kit, e.g. with a test strip of the test kit, upon which a test result is depicted, for example by means of a colorimetric method. The test kit can be used by healthcare professionals, untrained personnel and/or patients. An analysis of the identified biomarkers in a sample, such as nasal secretion sample, provides information about the status of epithelial cells in the nasal lumen, and provides detailed information about occurring symptoms as well as therapy success. The test kit, use, and methods of the invention are advantageous in that they can be used as point-of-care means for assessing allergic responses. In one embodiment, determining an allergic response is a part of a method of predicting and/or monitoring therapeutic success of an allergen specific immunotherapy.

The methods, kit, and use of the invention are advantageous in that no invasive sample is needed for analyzing allergic responses. Particularly, the methods, kit, and use of the invention are advantageous in that they involve a noninvasively obtained sample. Such involvement of noninvasively obtained samples allows for increasing patient compliance and comfort over methods, kits, and uses involving invasively or minimally invasively obtained samples.

The present invention provides diagnostic biomarkers and particularly a set of biomarkers that enable(s) monitoring of the inflammatory load of type-2 diseases such as allergies. In addition, the set of biomarkers allows to monitor and to predict the treatment success of allergen-specific immunotherapy. The set of biomarkers is used in the methods, kit, and use of the invention. These biomarker combinations are measured in nasal lining fluids, e.g. nasal secretion samples, which are obtained in a non-invasive manner. In one embodiment, the term "biomarker combination" and "set of biomarkers" is used interchangeably. The present invention is particularly useful for characterization of airway diseases, but also for point of care applications, where non-invasive sampling is important for the use in daily routine. An advantage of the methods, kit, and use of the invention is that non-invasively obtained samples can be used for said methods, kit, and use. Furthermore, the kit of the invention can be provided in the form of a self-test, e.g. for home care, that allows the patients to check their immune status, e.g. to get a first hint towards the origin of their airway symptoms, e.g. the allergens that they are allergic to, and the related airway disease. The set of biomarkers provides a valuable tool for the patient and his doctor to determine a patient's immune status, e.g. to predict and/or monitor therapeutic success of an allergen-specific immunotherapy and/or to determine an allergic response to an allergen. For example, the set of biomarkers can be used to navigate through the waves of seasonal exposure to allergens. Furthermore, the set of biomarkers allows to monitor the phases of a treatment. In one embodiment, for example by means of a personalized approach, the set of biomarkers can be used to study, manage, predict, monitor, prevent, determine, and/or diagnose allergic diseases.

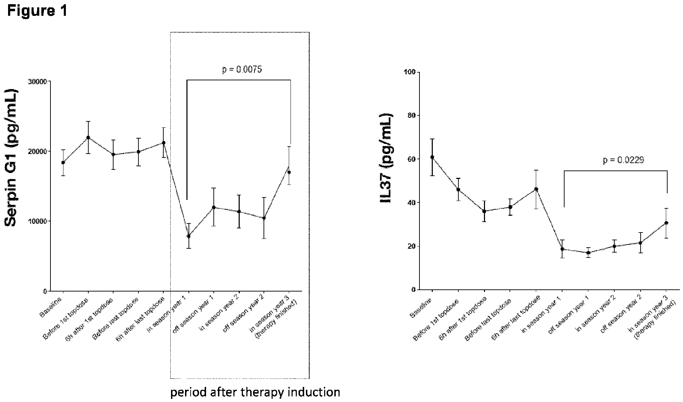

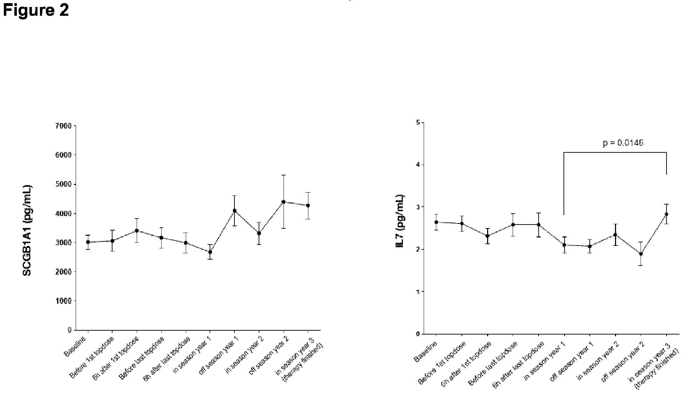

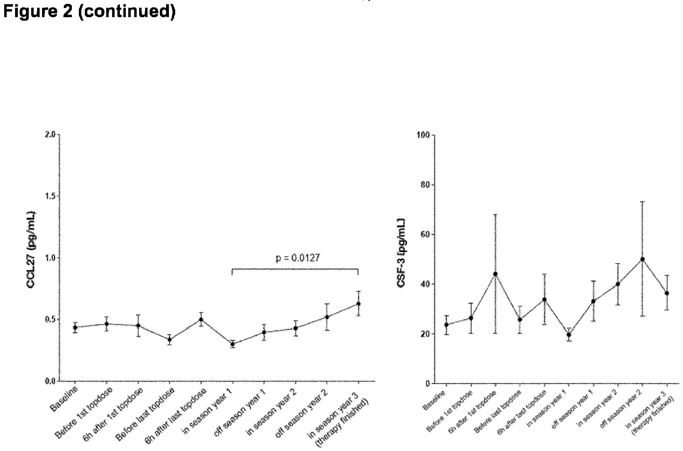

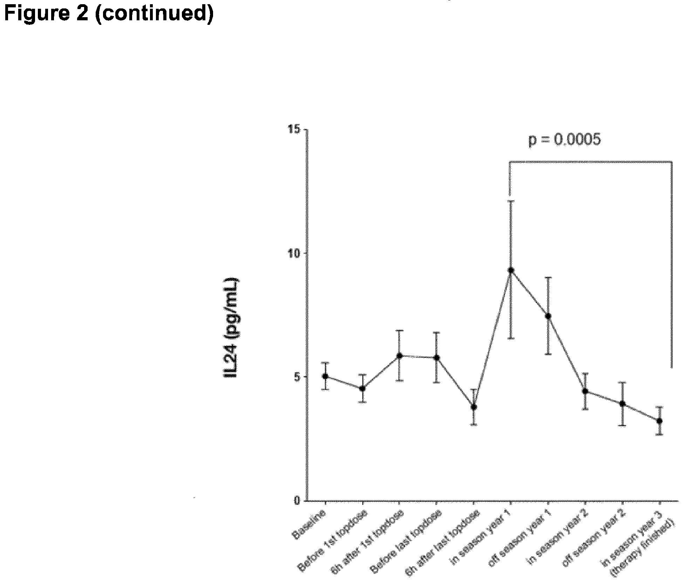

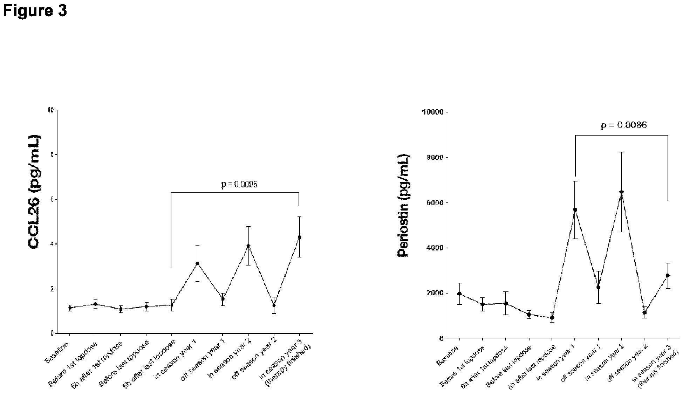

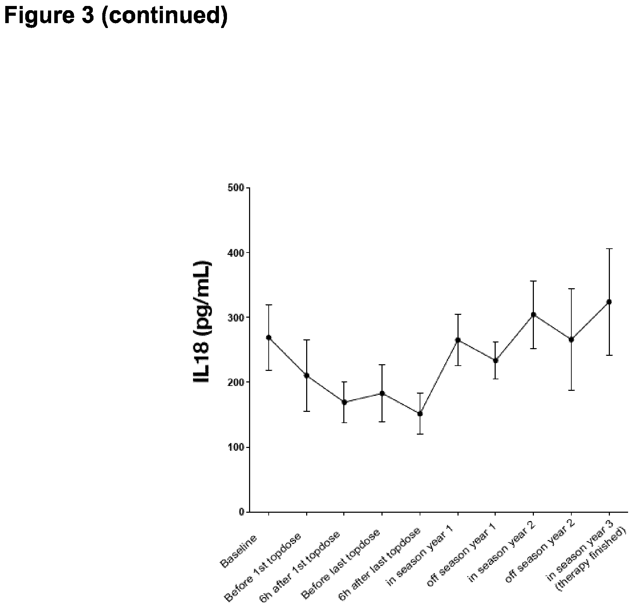

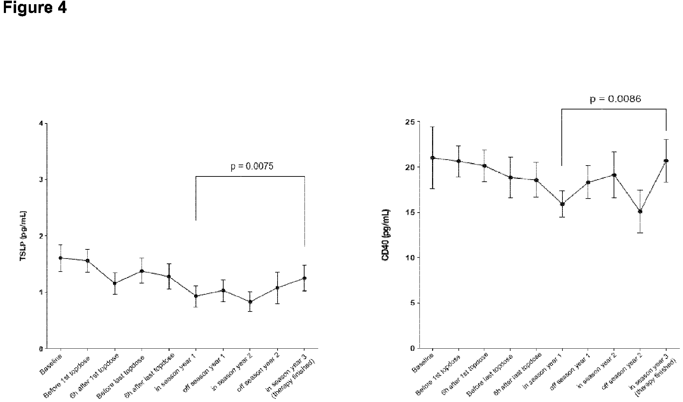

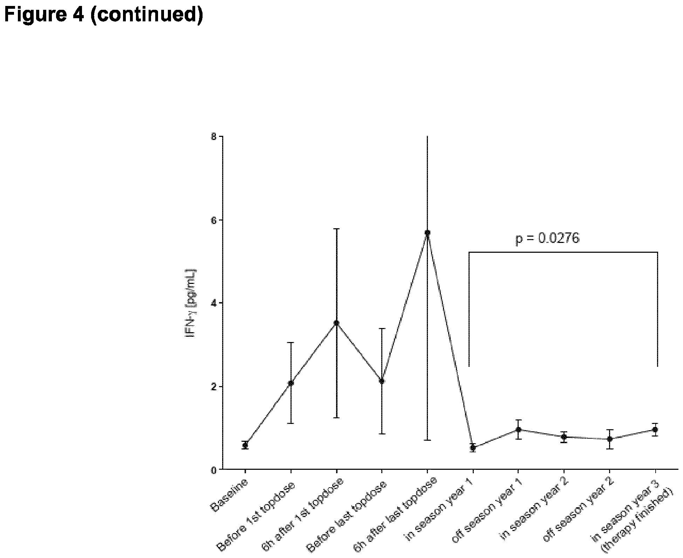

The present inventors identified a set of biomarkers by empirical means using transcriptome data of lower and upper airways. Several thousand genes were found to be differentially regulated in the transcriptomes. The present inventors further extended the analysis using nasal secretion measurements. The measured time courses revealed important epithelial responses that were grouped in the following way:
Dimension A): biomarkers of acute epithelial suppression (Fig. 1). The biomarkers Serpin G1 and IL-37 provide a subset of biomarkers for epithelial suppression and/or a process of convalescence. The suppression takes place at the level of epithelial cells that secrete into the airway lumen. Therefore, the identified biomarkers are, advantageously, measurable in non-invasive measurements. In one embodiment, the set of biomarkers comprises Serpin G1 and IL-37.
Dimension B): biomarkers of therapy amplitude (Fig. 2; SCGB1A1, IL7, CCL-27 and CSF-3) are increased upon allergen-specific immunotherapy and therefore allow for the monitoring of a treatment success. If these markers are not induced upon a therapeutic intervention, the dosing scheme and/or timing of the therapeutic intervention has/have to be modified. In one embodiment, the set of biomarkers comprises at least two of SCGB1A1, IL7, CCL-27, and CSF-3.
Dimension C): the selected biomarkers of epithelial seasonal response (POSTN, CCL26, and IL-18) diagnose the environmental response such as allergen season (Fig. 3). Biomarkers of basal stem cells and non-epithelial responses are two further control elements of cells that are plastic and depend on the infiltration (Fig. 4). In one embodiment, the set of biomarkers comprises at least two of POSTN, CCL26, and IL-18.

The invention provides a set of biomarkers that allows for a comprehensive monitoring of epithelial activity and therapeutic response, which is helpful in determining allergic responses, e.g. when predicting and/or monitoring therapeutic success of an AIT. Particularly, the set of biomarkers allows to determine an immunological status of the patient, e.g. for predicting and/or monitoring therapeutic success of an allergen-specific immunotherapy and/or for determining an allergic response to an allergen in a patient. In one embodiment, the set of biomarkers is a tool for the physician and/or the patient which allows a quantitative assessment on the condition of the airways, e.g., whether an allergic response is present or not.

The provided set of biomarkers is a highly advantageous tool for determining and/or assessing an overall immunological status. Furthermore, four different biological processes can be monitored and/or determined using a method, kit, or use involving the set of biomarkers. For example, the biomarker-based monitoring allows for quantifying an allergic inflammation, a seasonal impact, convalescence and/or therapeutic success. Particularly, such biomarker-based monitoring is highly useful in monitoring and/or predicting therapeutic success of an allergen specific immunotherapy.

In one embodiment, an allergen-specific immunotherapy comprises
i) an initial induction phase, wherein an allergen is repeatedly administered to a patient in increasing doses of said allergen up to a maximum dose effective to induce immunologic tolerance to said allergen in said patient, and
ii) a maintenance phase, wherein said allergen is administered repeatedly to said patient at said maximum dose,
and wherein, in said method, said biomarker level(s) of each biomarker of a set of biomarkers is determined in the first 1 - 7 weeks of the maintenance phase after the initial induction phase.

In one embodiment, said allergen-specific immunotherapy involves subcutaneous injection of an allergen, and comprises
i) an initial induction phase, where said allergen is repeatedly, preferably weekly, subcutaneously administered to a patient in increasing doses of said allergen up to a maximum dose effective to induce immunologic tolerance to said allergen in said patient, and
ii) a maintenance phase, wherein said allergen is subcutaneously administered repeatedly to said patient at said maximum dose,
wherein, preferably, said maintenance phase comprises
a first subphase ("top dose phase"), wherein said allergen is subcutaneously administered repeatedly at said maximum dose in a weekly or biweekly interval, and
a second subphase ("treatment maintenance phase"), wherein said allergen is subcutaneously administered repeatedly at the same maximum dose in intervals longer than during the top-dose phase, preferably every 4-6 weeks,
wherein said biomarker level(s) is/are determined 4-10 hours after administration of any maximum dose of said top-dose phase, preferably 4-10 hours after administration of the last maximum dose of said top-dose phase.

In one embodiment, said allergen-specific immunotherapy involves sublingual administration of an allergen and comprises
i) an initial induction phase, where said allergen is repeatedly, preferably every other day, sublingually administered to a patient in increasing doses of said allergen up to a maximum dose effective to induce immunologic tolerance to said allergen in said patient, and
ii) a maintenance phase, wherein said allergen is repeatedly, preferably daily, sublingually administered to said patient at said maximum dose,
wherein said biomarker level(s) is/are determined within the first 1 - 7 weeks of said maintenance phase, preferably 4-10 hours after administration of any maximum dose of said maintenance phase within said first 1 - 7 weeks of said maintenance phase.

The term "allergen-specific immunotherapy" (AIT), as used herein, is a therapy wherein an allergen is administered repeatedly to a patient over a defined period of time. Typically, such allergen-specific immunotherapy comprises an initial phase wherein an allergen is administered repeatedly in increasing doses to a patient, wherein such doses of allergen increase up to a maximum dose that is effective to induce immunologic tolerance to said allergen in said patient. Such initial phase is herein also sometimes referred to as "up-dosing phase" or "initial induction phase". The amount of the maximum dose that is effective to induce immunologic tolerance in a patient can be easily determined by a person skilled in the art. Such a maximum dose typically is the dose that is effective to induce immunologic tolerance to said allergen in a patient and is therefore herein also sometimes referred to as "effective dose". In practice, usually, every established immunotherapy vaccine will have a summary of product characteristics (SPC) with a recommended maintenance dose. However, an allergologist can also easily determine an individual patient's threshold(s) by updosing to a limit, where systemic reactions occur. Nowadays for any immunotherapy vaccine, every manufacturer usually conducts dosage-finding studies in which maximum doses are determined, and these are usually indicated in the SPC and the product leaflet and can be followed in practice.
Typically, there are different types of allergen-specific immunotherapies which differ from each other in terms of their mode of administration of the allergen. In one example, such allergen-specific immunotherapy involves subcutaneous administration, i.e. typically injection of an allergen. Such allergen-specific immunotherapy involving subcutaneous administration is also sometimes referred to as subcutaneous immunotherapy or subcutaneous allergen immunotherapy or SCIT. In another example, such allergen-specific immunotherapy involves sublingual administration of the allergen. Such allergen-specific immunotherapy involving sublingual administration is also sometimes referred to as sublingual immunotherapy or sublingual allergen immunotherapy or SLIT.
In either form of allergen-specific immunotherapy, there is comprised an initial induction phase, wherein an allergen is repeatedly administered to a patient in increasing doses of said allergen ("up-dosing phase") up to a maximum dose effective to induce immunologic tolerance to said allergen in said patient, and thereafter follows a maintenance phase in such allergen-specific immunotherapy, wherein said allergen is administered repeatedly to said patient at said maximum dose. In one embodiment of the method according to the present invention, said biomarker level(s) is/are determined in the first 1-7 weeks of the maintenance phase after the initial induction phase. In one embodiment, this applies to both SCIT and SLIT. Determination of biomarker levels is done by taking sample(s) from the patient as defined herein, and determining biomarker levels from such sample(s), particularly a biomarker level of each biomarker of a set of biomarkers.

In the subcutaneous immunotherapy, after the initial phase of increasing doses ("up-dosing phase") of allergen up to a maximum dose (during which initial phase the allergen is administered repeatedly, preferably weekly), typically there is a maintenance phase thereafter, wherein the allergen is administered repeatedly at the maximum dose. Typically, in such subcutaneous immunotherapy, the allergen is administered subcutaneously, preferably by way of an injection, of a solution or dispersion. Injections are preferably administered subcutaneously.

In one embodiment of such subcutaneous immunotherapy, the maintenance phase can be subdivided further into a first sub-phase, herein also sometimes referred to as "top-dose phase", wherein said allergen is administered at said maximum dose in defined intervals, preferably weekly or biweekly. Subsequently, there is a second subphase, herein also sometimes referred to as "treatment maintenance phase", wherein said allergen is administered repeatedly at the same maximum dose as in the top-dose phase, but in intervals longer than during the top-dose phase, preferably every 4-6 weeks. Hence, in one embodiment of the method of predicting and/or monitoring therapeutic success of an allergen-specific immunotherapy (AIT) in a patient according to the present invention, an allergen-specific immunotherapy involves a subcutaneous administration of allergen to a patient and comprises an initial induction phase of increasing doses of allergen administered weekly, a top-dose phase of maximum doses of allergen administered weekly or biweekly, and a treatment maintenance phase of maximum doses of allergen administered every 4-6 weeks. In such embodiments, the initial phase typically is for a period of 3-8 weeks, the top-dose phase is for a period of 1-4 weeks, and the treatment maintenance phase is for 1-3 years. In one embodiment, the initial phase may also be shortened due to the employment of an accelerated administration schedule (aka "rush scheme") in which either the frequency of the injections or the incremental dose increase between consecutive injections or both may be increased. Thus, accelerated schedules either involve administering more injections per visit/administration, increasing the dose more between consecutive injections, or both. If such an accelerated scheme is employed, the initial induction phase has a length of approximately 1 - 2 weeks, and the frequency of administration during such initial induction phase may be changed from weekly to daily or even shorter time intervals.

Preferably, in an embodiment of subcutaneous immunotherapy, comprising an initial induction phase of increasing doses of allergen administered weekly, a top-dose phase of maximum doses of allergen administered weekly or biweekly, and a treatment maintenance phase of maximum doses of allergen administered every 4-6 weeks, said biomarker level of each biomarker of a set of biomarkers is determined after administration of any maximum dose of said top-dose phase, preferably after administration of the last maximum dose of said top-dose phase. In one such embodiment, said biomarker level of each biomarker of a set of biomarkers is determined 4 - 10 hours after administration of any maximum dose of said top-dose phase, preferably 4 - 10 hours after administration of the last maximum dose of said top-dose phase. In one embodiment, when referring to a biomarker level being determined, said determination comprises determining a biomarker level of each biomarker of a set of biomarkers.

In the sublingual immunotherapy (SLIT), after the initial phase of increasing doses ("up-dosing phase") of allergen up to a maximum dose, typically, there is a maintenance phase thereafter, wherein the allergen is administered repeatedly at the maximum dose. Typically, in such sublingual immunotherapy, the allergen is administered sublingually as a solution or dispersion. Drops are orally administered and may be kept by a patient sublingually ("under the tongue") for a defined period of time, e.g. 1-10 minutes, preferably 1 - 5 minutes, more preferably 1 - 3 minutes.

In one embodiment of such sublingual immunotherapy, in the initial induction phase, an allergen is repeatedly, preferably every other day, sublingually administered to a patient in increasing doses of said allergen up to a maximum dose effective to induce immunologic tolerance to said allergen in said patient, in the subsequent maintenance phase, said allergen is repeatedly, preferably daily, sublingually administered to said patient at said maximum dose. Hence, in one embodiment of the method of predicting and/or monitoring therapeutic success of an allergen-specific immunotherapy (AIT) in a patient according to the present invention, an allergen-specific immunotherapy involves a sublingual administration of allergen to a patient and comprises an initial induction phase of increasing doses of allergen administered every other day, and a maintenance phase of maximum doses of allergen administered daily. In such embodiments, the initial induction phase of increasing doses typically is for a period of 3-6 weeks, and the maintenance phase of maximum doses typically is for a period of 10-18 months. In one embodiment the total duration of the sublingual immunotherapy is 12-18 months, and the treatment schedule comprises a six-week up-dosing phase, involving administration of the allergen every other day, followed by a daily maintenance treatment during the maintenance phase. In one example of such SLIT, up-dosing involves three administrations of allergen per week, with increasing doses from week 1 to week 6 (as an example e.g. one drop of allergen solution administered per administration in week 1, two drops of allergen solution administered per administration in week 2, 4 drops of allergen solution administered per administration in week 3, and 7, 10 and 14 drops of allergen solution administered per administration in weeks 4, 5, and 6, respectively). In one embodiment of such SLIT, the maintenance phase involves a daily administration of the maximum dose, which had been achieved in the last week of up-dosing, as a maintenance dose. Preferably, in an embodiment of sublingual immunotherapy, comprising an initial induction phase of increasing doses of allergen administered weekly, and a maintenance phase of maximum doses of allergen administered daily, said biomarker level of each biomarker of a set of biomarkers is determined after administration of any maximum dose of said maintenance phase during the first 1 - 7 weeks after the initial induction phase.

In one embodiment, AIT is timed such that the initial induction phase and the top-dose phase (in the case of SCIT), or the initial induction phase and the first 1 - 7 weeks of the maintenance phase (in the case of SLIT) are performed before the respective allergen occurs naturally in the environment, e.g. before the allergen is distributed naturally by its natural source, e.g. before pollination season. For example, if the allergen is birch pollen, AIT is performed such that the induction phase and the top-dose phase (in the case of SCIT), or the initial induction phase and the first 1 - 7 weeks of the maintenance phase (in the case of SLIT) are performed such that they are finished before birch pollen is distributed by birch trees in their natural habitat. In one embodiment, "off season" relates to a season in which pollen is not distributed in a substantial amount in the air, such as prior to or after pollen is distributed by the respective plants. In one embodiment, "in season" relates to a season in which pollen is distributed in a substantial amount in the air, such as during a season in which pollen is distributed by the respective plants.

In one embodiment of the method of predicting and/or monitoring therapeutic success of an allergen-specific immunotherapy (AIT) in a patient according to the present invention involving a SCIT scheme, the determination of said biomarker level of each biomarker of a set of biomarkers is done after administration of any maximum dose in the top-dose phase, preferably after administration of the last maximum dose in the top-dose phase. The inventors have found that whilst such biomarker levels a set of biomarkers seems to be of general value for predicting therapeutic success of an allergen specific immunotherapy (AIT), the determination after administration of any maximum dose in the top-dose phase is particularly suitable for predicting therapeutic success of the allergen-specific immunotherapy. The inventors have found that it is particularly useful for predicting therapeutic success if such determination is performed within 2 - 12 hours, preferably within 4 - 10 hours after the respective maximum dose has been administered.

In one embodiment of the method of predicting and/or monitoring therapeutic success of an allergen-specific immunotherapy (AIT) in a patient according to the present invention involving a SLIT scheme, the determination of said biomarker level of each biomarker of a set of biomarkers is done after administration of any maximum dose in the maintenance phase within the first 1 - 7 weeks of such maintenance phase, preferably after administration of any maximum dose in weeks 4 - 7 of such maintenance phase. The inventors have found that it is particularly useful for predicting therapeutic success if such determination is performed within 2 - 12 hours, preferably within 4-10 hours after the respective maximum dose has been administered.

The term "therapeutic success", as used herein is meant to refer to a state, wherein the symptoms of the allergic disease are reduced or absent in comparison to an allergen-exposed patient's status prior to said allergen-specific immunotherapy. In one preferred embodiment, therapeutic success is determined by a patient-assessed retrospective assessment of seasonal allergic symptoms (RAAS) by scoring overall disease symptoms, e. g. hay fever symptoms, in comparison to the season before the start of immunotherapy and in year 3 in comparison to the time prior to treatment. The score given by the patient is on a scale between +3 ("much better") to 0 ("no change") to -3 ("much worse"). Determination or measurement of therapeutic success by RAAS is known to a person skilled in the art and can for example be performed as described in A.M. Chaker et al., 2016, Journal of Allergy and Clinical Immunology, 137, pp.452-461. In another preferred embodiment, therapeutic success is determined or measured by a combined symptom medication score (CSMS) as e.g. described in Pfaar et al. 2014, Allergy; 69, pp.854-867, or by visual analog scale (VAS) as e.g. described in Pfaar et al. 2014, Allergy; 69, pp.854-867, making use of exposure chambers.

The term "patient", as used herein, relates to a human or an animal. In one embodiment, said patient is a patient suffering from an allergic disease and/or a patient having a disposition to develop an allergic disease. As used herein, the term "a patient suffering from an allergic disease" is meant to refer to a patient who, upon exposure to an allergen, develops or has developed symptoms of said allergic disease; but such term should also be understood as including a patient who, prior to exposure to said allergen, is nevertheless likely or prone to develop such symptoms upon exposure to the allergen. This can for example be, because it is clear from a patient's history that the patient has had attacks of such allergic disease in earlier times, for example during the flowering period of specific plants in previous year(s) or seasons. The term is also meant to include patients who have a known disposition to develop symptoms of an allergic disease or a family history of symptoms of an allergic disease, but have not yet developed such disease in the present allergen season.

The term "allergic disease", as used herein, relates a disease involving an allergic immune response, e.g. a hypersensitivity of the immune system to typically harmless substances, for example hay fever, allergic rhinitis, allergic asthma, allergic conjunctivitis, food allergy, or stinging insect hypersensitivity. In one embodiment, an allergic disease is allergic rhinitis, bee sting allergy, a food allergy, hay fever, or allergic asthma.

The term "allergen", as used herein, is meant to refer to a wide range of substances or agents, such as plant pollen, in particular tree pollen, such as from birch, alder, poplar, elm, willow, oak, maple, ash, hazel, and beech, grass pollen (Poaceae), such as pollen from species like Phleum pratense, Lolium perenne, Dactylis glomerata, Holcus lanatus, Poa pratensis, Festuca pratensis, Cynodon dactylon, Paspalum notatum, Anthoxanthum odoratum and rye (secale cereale), weed pollen, such as from nettle(s), ragweed, mugwort, plantain other airborne particles, such as mould spores, mites, animal dander, fungi and fungal spores, but also venom from insects such as bees or wasps. As an example, as allergen(s), a mixture of grass pollen can be used, e.g. pollen from Holcus lanatus, Dactylis glomerata, Lolium perenne, Phleum pratense, Poa pratensis, and Festuca pratensis. In one embodiment, said allergen is any allergen involved in any allergic disease, such as allergic rhinitis, bee sting allergy, food allergies, hay fever, and allergic asthma, for example food allergens, pollen, e.g. grass pollen, tree pollen and weed pollen, other airborne allergens, such as fungi, fungal spores, dust, mites, and animal dander. Numerous preparations of allergens are commercially available, e.g. Allergovit^{®} 6-grasses (Allergopharma GmbH & Co. KG, Reinbek, Germany), a 100 % mixture of allergens from 6 grass pollen species (Holcus lanatus, Dactylis glomerata, Lolium perenne, Phleum pratense, Poa pratensis, and Festuca pratensis) chemically modified with formaldehyde to produce an allergoid, which is then coprecipitated with aluminium hydroxide, was used. Allergovit^{®} 6-grasses is specified in therapeutic units per mL (TU/mL), provided in two strengths (A: 1000 TU/mL and B: 10,000 TU/mL).

The present inventors have found that if the biomarker levels of a first biomarker collection of a set of biomarkers are normalized in a second sample compared to a first sample, it is likely that an allergen-specific immunotherapy will be successful in such patient. If the biomarker levels of a first biomarker collection of a set of biomarkers are not normalized in a second sample compared to a first sample, it is likely that an allergen-specific immunotherapy will not be successful in said patient. Because, in a preferred embodiment, the biomarker level of each biomarker of a set of biomarkers is determined relatively early during the allergen-specific immunotherapy, namely during the induction phase with increasing doses or during the first 2-8 weeks of the subsequent maintenance phase, for example after administration of the last maximum dose during the top-dose phase, it can then already be decided whether or not to continue with such allergen-specific immunotherapy, based on a prediction of therapeutic success using the aforementioned biomarker levels. In one embodiment, the biomarker levels are determined in one or several samples obtained from the patient. In one embodiment, a first sample of the patient is not needed as the biomarker level of such first sample can be replaced using a reference value and/or reference sample of a healthy individual. In one embodiment, in the methods of the present invention, a first sample of the patient is not needed since a reference value and/or reference sample of a healthy individual can be used instead of a first sample, e.g. as a baseline for determining whether a biomarker level in the second sample is normalized and/or abnormal.

In one embodiment, the method of predicting and/or monitoring therapeutic success of an allergen-specific immunotherapy in a patient is an ex vivo and/or in vitro method. In one embodiment, the allergen-specific immunotherapy (AIT) and/or an administration of an allergen is/are not part of the method. In one embodiment, the method of determining an allergic response to an allergen in a patient is an ex vivo and/or in vitro method. In one embodiment, an allergen-specific immunotherapy (AIT) and/or an administration of an allergen is/are not part of the method. In one embodiment, the methods of the invention and the use of the invention are not practiced on the human (or animal) body and are performed in vitro and/or ex vivo, for example using samples obtained from a patient. In one embodiment, a patient is a human or an animal e.g. a non-human mammal. In one embodiment, said patient is a patient for whom therapeutic success or non-success of an allergen-specific immunotherapy is to be predicted and/or monitored. In one embodiment, said patient is a patient for whom an allergic response is to be determined.

In one embodiment, the term "predicting and/or monitoring therapeutic success", as used herein, relates to assessing whether an allergen-specific immunotherapy is and/or will be successful in a patient. In one embodiment, such assessment is performed prior to an allergen-specific immunotherapy (AIT) for predicting therapeutic success of such AIT. In one embodiment, such assessment is performed during an AIT for monitoring therapeutic success of such AIT. In one embodiment, such assessment is performed prior to and during an AIT.

The term "first sample", as used herein, relates to a sample of patient obtained at a first time point, e.g. prior to an allergen exposure. In one embodiment, such allergen exposure is any exposure to an allergen, such as a season-based allergen exposure, e.g. exposure to pollen in the air during pollen season, an environment-based allergen exposure, e.g. fungal spores, dust, or mites occurring in a household, an allergen exposure deriving from an administration of an allergen, e.g. an oral, nasal, subcutaneous, or dermal administration, such as an administration for an AIT. In one embodiment, a first sample is obtained at a time point prior to an AIT and/or at an earlier time point during an AIT which is earlier than a second time point during said AIT. In one embodiment, a first sample is a sample obtained prior to and/or at the beginning of an AIT, either seasonal or out of season, and such first sample provides a baseline, i.e. a reference value and/or reference sample. In one embodiment, a first sample, i.e. an earlier sample of a patient, is used as a baseline, e.g. a sample obtained prior to an allergen exposure or prior to a further allergen exposure of repeated allergen exposures, e.g. repeated allergen exposures during an AIT.

The term "first time point", as used herein, is a time point which is prior to a second time point. In one embodiment, said first time point is a time point prior to an AIT or during an AIT, wherein said first time point is prior to a second time point prior to or during said AIT. In one embodiment, said first time point is a time point prior to an allergen exposure, such as a first allergen exposure or a further allergen exposure. In one embodiment, said first time point is time point prior to allergen exposure prior to or during an AIT.

The term "second sample", as used herein, relates to a sample of a patient obtained at a second time point, e.g. after an allergen exposure. In one embodiment, a second sample is obtained at a time point later than a first time point, e.g. prior to or during an AIT. The term "second time point", as used herein, is a time point after a first time point. In one embodiment, said second time point is after an allergen exposure, e.g. an allergen exposure during an AIT. In one embodiment, a first sample of a patient is obtained at a first time point prior to an allergen exposure, e.g. prior to a first or further allergen exposure, and a second sample of said patient is obtained at a second time point after said allergen exposure.

In one embodiment, the term "allergen exposure", as used herein, relates to a patient or healthy individual being exposed to an allergen. In one embodiment, the term "first or further allergen exposure" is meant to refer to an allergen exposure which is the first allergen exposure or a further (i.e. one of several allergen exposures) allergen exposure. For example, a further allergen exposure is one of several allergen exposures during an AIT. In one embodiment, an allergen exposure relates to any exposure to an allergen, such as an active administration of an allergen or a passive exposure due to the environment. For example, an allergen exposure may be a season-based allergen exposure, e.g. exposure to pollen in the air during pollen season, an environment-based allergen exposure, e.g. fungal spores, dust, or mites occurring in a household, or an allergen exposure deriving from an administration of an allergen, e.g. an oral, nasal, subcutaneous, or dermal administration, such as an administration for an AIT. In one embodiment, an allergen exposure is an environmental allergen exposure, e.g. pollen in the air. In one embodiment, an allergen exposure is an administration of an allergen to a patient, e.g., a sublingual or subcutaneous administration.

The term "determining a biomarker level", as used herein, relates to determining the level of a biomarker using any measurement method and/or analytical technique known to person skilled in the art, e.g. analyzing and/or measuring a biomarker level such as a protein level and/or nucleic acid level. In one embodiment, a biomarker level is determined by determining a protein level and/or a nucleic acid level, preferably a protein level, of each biomarker of a set of biomarkers in a sample. In one embodiment, the biomarker levels are quantitatively determined using any method known to a person skilled in the art, such as sandwich ELISA. In one embodiment, a means to perform a quantitative determination of biomarker levels in a sample is a means to perform a high-performance liquid chromatography (HPLC), liquid chromatography-mass spectrometry (LC/MS), enzyme-linked immunosorbent assay (ELISA), protein immunoprecipitation, immunoelectrophoresis, Western blot, protein immunostaining, microarray, PCR such as real time PCR, next generation sequencing, transcriptome-based assays, or a capillary test. In one embodiment, a biomarker level is determined using a method selected from high-performance liquid chromatography (HPLC), liquid chromatography-mass spectrometry (LC/MS), enzyme-linked immunosorbent assay (ELISA), protein immunoprecipitation, immunoelectrophoresis, Western blot, protein immunostaining, microarray, PCR such as real time PCR, next generation sequencing, transcriptome-based assays, and capillary test systems. In one embodiment, a means to perform a quantitative determination of a biomarker level in a sample is used to determine a biomarker level.

In one embodiment, the term "biomarker level", as used herein, relates to a relative and/or absolute amount of a biomarker in a sample, e.g. the relative and/or absolute amount of a biomarker in the form of a protein and/or in the form of a nucleic acid. In one embodiment, a biomarker level is a quantitative measure of a specific biomarker. In one embodiment, a biomarker level allows to determine whether an allergic response is present or not and/or allows to predict whether an AIT is likely to be successful.

The term "set of biomarkers", as used herein, relates to a group of biomarkers useful in analyzing an allergic response, e.g. for determining whether an allergic response is present or not and whether an allergic response is likely to occur. For example, a set of biomarkers is useful in predicting and/or monitoring therapeutic success of an AIT and for determining an allergic response to an allergen in a patient. Particularly, the specific combination of at least two, preferably at least three, more preferably at least four, even more preferably at least five biomarkers selected from a first biomarker collection consisting of SCGB1A1, IL-7, Serpin Gi, IL-37, CSF-3, NOG, CCL-27, IL-24, POSTN, CCL26, and IL-18 allows to analyze whether an allergic response occurs in response to an allergen, for example during an allergen-specific immunotherapy. In one embodiment, a method of predicting and/or monitoring therapeutic success of an allergen-specific immunotherapy (AIT) of the invention comprises a method of determining an allergic response to an allergen of the invention. In one embodiment, a method of determining an allergic response to an allergen of the invention comprises a method of predicting and/or monitoring therapeutic success of an allergen-specific immunotherapy (AIT) of the invention. Furthermore, the set of biomarkers allows to examine the extent of an allergic response in a quantitative manner. In one embodiment, the biomarker levels of the biomarkers selected from the first biomarker collection are standardized using one or more biomarkers selected from a second biomarker collection consisting of CCL20, CCL23, CSF-1, AREG, TSLP, CD40, IFN-γ, IL-4, IL-13, and IL-5. In one embodiment, the first biomarker collection comprises anti-inflammatory biomarkers and pro-inflammatory biomarkers. In one embodiment, the first biomarker collection consisting of SCGB1A1, IL-7, Serpin Gi, IL-37, CSF-3, NOG, CCL-27, IL-24, POSTN, CCL26, and IL-18 comprises anti-inflammatory biomarkers selected from SCGB1A1, IL-7, Serpin Gi, IL-37, and NOG, and pro-inflammatory biomarkers selected from CCL-27, IL-24, POSTN, CCL26, CSF-3, and IL-18. In one embodiment, the second biomarker collection comprises standardizing biomarkers. In one embodiment, the set of biomarkers comprises biomarkers of the first biomarker collection and optionally further comprises biomarkers of the second biomarker collection.

In one embodiment, the set of biomarkers comprises two or more biomarkers of a first biomarker collection, and optionally further comprises one or more biomarkers of a second biomarker collection. In one embodiment, the first biomarker collection consists of SCGB1A1, IL-7, Serpin Gi, IL-37, CSF-3, NOG, CCL-27, IL-24, POSTN, CCL26, and IL-18. In one embodiment, the second biomarker collection consists of CCL20, CCL23, CSF-1, AREG, TSLP, CD40, IFN-γ, IL-4, IL-13, and IL-5. In one embodiment, the set of biomarkers comprises at least two, preferably at least three, more preferably at least four, even more preferably at least five, even more preferably at least six, seven, eight, nine, ten or eleven biomarkers selected from a first biomarker collection consisting of SCGB1A1, IL-7, Serpin Gi, IL-37, CSF-3, NOG, CCL-27, IL-24, POSTN, CCL26, and IL-18. In one embodiment, the set of biomarkers comprises all biomarkers of the first biomarker collection. In one embodiment, the set of biomarkers further comprises one or more biomarkers, e.g. one, two, three, four, five, six, seven, eight, nine or ten biomarkers, selected from the second biomarker collection consisting of CCL20, CCL23, CSF-1, AREG, TSLP, CD40, IFN-γ, IL-4, IL-13, and IL-5. In one embodiment, the set of biomarkers further comprises all biomarkers of the second biomarker collection. In one embodiment, the set of biomarkers comprises biomarkers SCGB1A1, CCL-27, Serpin Gi, IL-37, and IL-24. In one embodiment, the set of biomarkers comprises biomarkers SCGB1A1, CCL-27, Serpin Gi, IL-37, and IL-24, and optionally further comprises at least one standardizing biomarker, i.e. a biomarker of the second biomarker collection.

The term "first biomarker collection", as used herein, relates to a collection of biomarkers comprising anti-inflammatory biomarkers and pro-inflammatory biomarkers. In one embodiment, a set of biomarkers comprising biomarkers of a first biomarker collection is useful in determining whether an allergic response is present, absent, and/or is likely to occur. The term "second biomarker collection", as used herein, relates to a collection of biomarkers comprising standardizing biomarkers. In one embodiment, such second biomarker collection is useful in standardizing biomarker levels of biomarkers of a first biomarker collection. In one embodiment, biomarker levels of biomarkers of the second biomarker collection are used as an internal control for biomarker levels. Such internal control can be used to standardize samples and measurements, e.g. by reducing measurement artifacts resulting from seasonal variation, sample variation, and/or patient variation. In one embodiment, biomarker levels of a second biomarker collection allow to increase the reliability of comparisons between a first sample and a second sample. A set of biomarkers further comprising at least one biomarker of a second biomarker collection is advantageous in that inter-sample variation is reduced.

The term "normalization", as used herein, relates to a biomarker level becoming or being "normal", i.e. a biomarker level becoming or being a biomarker level that corresponds to a biomarker level of a healthy individual. When referring to a "biomarker level becoming normal", such term is meant to refer to the biomarker level, in the second sample, having a value that is closer to a reference value and/or reference sample than the biomarker level in the first sample. In one embodiment, a normalization is an approximation to and/or attainment of a reference value for the respective biomarker level, preferably a reference value of a healthy individual, and/or is an approximation to and/or attainment of a respective biomarker level determined in a reference sample, preferably a reference sample of a healthy individual. In one embodiment, the term "an approximation" is meant to refer to a biomarker level becoming normal, i.e. the biomarker level in a second sample being closer to a reference value and/or reference sample than the biomarker level in a first sample. In one embodiment, the term "attainment" is meant to refer to the biomarker level having become normal. In one embodiment, a "normal" biomarker level is the biomarker level of a healthy and/or convalesced individual. In one embodiment, a reference value and reference sample relate to a value and sample, respectively, of a healthy and/or convalesced individual. In one embodiment, the term "healthy individual" includes convalesced individuals. In one embodiment, the biomarker levels of a first biomarker collection are normalized in a second sample compared to a first sample, i.e. the biomarker levels in the second sample are closer to respective reference biomarker levels than the biomarker levels in the first sample, which indicates that an AIT is likely to be successful. The term "reference biomarker levels" includes reference values of healthy individuals and biomarker levels determined in reference samples of healthy individuals.

In one embodiment, a set of biomarkers comprises at least one anti-inflammatory biomarker, e.g. SCGB1A1, IL-7, Serpin Gi, IL-37, and/or NOG. In such case of an anti-inflammatory biomarker, a "normalization" of the biomarker level relates to an increase in the biomarker level of the anti-inflammatory biomarker. If an AIT is successful or is likely to be successful, the biomarker level of an anti-inflammatory biomarker becomes normal, i.e. is increased in a second sample compared to first sample, preferably is increased to a biomarker level that corresponds to a reference biomarker level of a healthy individual. In one embodiment, a set of biomarkers comprises at least one pro-inflammatory biomarker, e.g. CCL-27, IL-24, POSTN, CCL26, CSF-3, and/or IL-18. In such case of a pro-inflammatory biomarker, a "normalization" of the biomarker level relates to a decrease in the biomarker level of the pro-inflammatory biomarker. If an AIT is successful or is likely to be successful, the biomarker level of the pro-inflammatory biomarker becomes normal, i.e. is decreased, preferably is decreased to a reference biomarker level of a healthy individual. In one embodiment, such increase or decrease is measurable in a second sample compared to a first sample, a third sample compared to a second or first sample, or any further sample compared to a previous sample. In one embodiment, the methods of the invention comprise providing more than two samples, for example, three samples, four samples, five samples, six samples, or more. In one embodiment, any sample obtained at a simultaneous or later, preferably later, time point than a first sample can be considered as "second sample", and any sample obtained at a simultaneous or earlier, preferably earlier, time point than a second sample can be considered as "first sample".

The term "determining an allergic response to an allergen in a patient", as used herein, relates to determining whether an allergic response is present, absent, or is likely to occur. In one embodiment, such determination of an allergic response is useful in determining whether a patient is allergic to an allergen. In one embodiment, such determination of an allergic response is useful in determining whether an allergic response successfully decreases during the course of an AIT. The methods of the invention are highly advantageous in that allergic responses can be determined in a noninvasively obtained sample. Furthermore, methods of the invention are highly advantageous in that allergic responses can be tested in vitro/ex vivo. In one embodiment, a method of determining an allergic response may be used for measuring an allergic response during an AIT. In one embodiment, a method of predicting and/or monitoring therapeutic success of an AIT may comprise a method of determining an allergic response to an allergen in a patient. For example, using a method of determining an allergic response to an allergen in a patient of the invention, it is possible to measure the allergic response to an allergen exposure during an AIT. Furthermore, it is possible to compare the allergic responses in different treatment cycles of an AIT to monitor whether the allergic responses decrease in response to the treatment or not. In one embodiment, a method of determining an allergic response involves determining a biomarker level in one or more samples of a patient, e.g. in one sample, wherein a reference value and/or reference sample is used as a baseline, or in a first and second sample, wherein a first sample is used as a baseline.

In one embodiment, the term "abnormal", as used herein, relates to a biomarker level not being "normal", i.e. a biomarker level not being and/or corresponding to the biomarker level of a healthy individual. In one embodiment, a "healthy individual" is an individual not having an allergic response to an allergen. In one embodiment, when referring to a reference value and/or reference sample of a healthy individual, such individual does not have an allergic response to the specific allergen targeted by an AIT and/or the allergen of a respective allergen exposure.

In one embodiment, an allergic response is present if a biomarker level of one or more biomarkers of a first biomarker collection is abnormal in a second sample (obtained after allergen exposure) compared to a first sample (obtained prior to said allergen exposure). In one embodiment, a biomarker level is "abnormal" if the biomarker level deviates from a reference value for the respective biomarker level, preferably a reference value of a healthy individual, and/or if said biomarker level deviates from a respective biomarker level determined in a reference sample, preferably a reference sample of a healthy individual. In one embodiment, a biomarker level is "abnormal" if the biomarker level determined in a second sample obtained after an allergen exposure deviates from a biomarker level determined in a first sample obtained prior to said allergen exposure. In one embodiment, the biomarker levels of a first biomarker collection are abnormal in a second sample compared to a first sample, which indicates that an allergic response is present or is likely to occur.

In one embodiment, a set of biomarkers comprises at least one anti-inflammatory biomarker; in such case of an anti-inflammatory biomarker, the anti-inflammatory biomarker level being "abnormal" relates to a decrease in the biomarker level of the anti-inflammatory biomarker compared to a reference value, reference sample, and/or first sample. In one embodiment, the reference sample is a first sample, optionally obtained prior to an allergen exposure. In one embodiment, the biomarker level being abnormal relates to a decrease in the biomarker level of at least one anti-inflammatory biomarker in said sample compared to a reference value, reference sample, and/or first sample. In one embodiment, a set of biomarkers comprises at least one pro-inflammatory biomarker; in such case of a pro-inflammatory biomarker, the pro-inflammatory biomarker level being "abnormal" relates to an increase in the biomarker level of the pro-inflammatory biomarker compared to a reference value, reference sample, and/or first sample. In one embodiment, the biomarker level being abnormal relates to an increase in the biomarker level of at least one pro-inflammatory biomarker in a sample compared to a reference value, reference sample, and/or first sample. In one embodiment, a reference sample is a first sample obtained prior to an allergen exposure, e.g. prior to a first or further allergen exposure. In one embodiment, such decrease or increase is measurable in a second sample compared to a first sample, a third sample compared to a second or first sample, or any further sample compared to a previous sample. In one embodiment, a reference value and/or reference sample provide a reference, i.e. baseline, for the respective biomarker level, preferably the respective biomarker level of a healthy individual ("normal biomarker level").

In one embodiment, the term "standardizing a biomarker level", as used herein, relates to using an internal control to reliably determine biomarker levels. Such internal control can be a standardizing biomarker, for example, a gene or gene product of housekeeping gene, and/or any biomarker of the second biomarker collection, in particular CCL20, CCL23, CSF-1, AREG, TSLP, CD40, IFN-γ, IL-4, IL-13, and/or IL-5. By standardizing a biomarker level of a biomarker of the first biomarker collection using a biomarker level of a biomarker of the second biomarker collection, inter-sample, inter-individual, and inter-measurement variation can be reduced. In one embodiment, said biomarker level of one or more biomarkers of said biomarkers selected from said first biomarker collection is/are standardized using a biomarker level of one or more biomarkers selected from said second biomarker collection. The term "standardizing biomarker", as used herein, relates to a biomarker useful for standardizing biomarker levels, particularly a biomarker such as CCL20, CCL23, CSF-1, AREG, TSLP, CD40, IFN-γ, IL-4, IL-13, and IL-5. In one embodiment, the biomarker level of such standardizing biomarker does not increase or decrease in response to an allergen. In one embodiment, the expression of such standardizing biomarker is stable. Particularly, such level of a standardizing biomarker does not increase or decrease in response to an allergen in a patient suffering from allergic disease and neither in a healthy individual.

The term "anti-inflammatory biomarker", as used herein, relates to a biomarker coinciding with an anti-inflammatory response, e.g. contributing to an anti-inflammatory response and/or being associated with an anti-inflammatory response. In one embodiment, an anti-inflammatory biomarker is selected from SCGB1A1, IL-7, Serpin Gi, IL-37, and NOG, preferably selected from SCGB1A1, Serpin Gi, and IL-37. In one embodiment, a normalization of an anti-inflammatory biomarker level comprises an increase in the biomarker level of the anti-inflammatory biomarker in a second sample compared to a first sample. In one embodiment, an anti-inflammatory biomarker level becoming or being abnormal comprises a decrease in the biomarker level of the anti-inflammatory biomarker in a second sample compared to a first sample.

The term "pro-inflammatory biomarker", as used herein, relates to a biomarker coinciding with a pro-inflammatory response, e.g. contributing to a pro-inflammatory response and/or being associated with a pro-inflammatory response. In one embodiment, a pro-inflammatory biomarker is selected from CCL-27, IL-24, POSTN, CCL26, CSF-3, and IL-18, preferably selected from CCL-27, and IL-24. In one embodiment, a normalization of a pro-inflammatory biomarker level comprises a decrease in the biomarker level of the pro-inflammatory biomarker in a second sample compared to a first sample. In one embodiment, a pro-inflammatory biomarker level becoming or being abnormal comprises an increase in the biomarker level of the pro-inflammatory biomarker in a second sample compared to a first sample.

In one embodiment, the term "sample", as used herein, relates to a sample of a patient or of a healthy/convalesced individual. In one embodiment, a sample is a noninvasive sample. In one embodiment, a sample is a sample noninvasively obtained from a patient, preferably is a noninvasively obtained nasal secretion sample. In one embodiment, a noninvasively obtained sample, preferably a nasal secretion sample, is different from a nasal scraping, since said nasal scraping is minimally invasive. In particular, providing a nasal scraping is minimally invasive whereas providing a noninvasively obtained sample is noninvasive. In one embodiment, the nasal secretion is obtained using a nanosorption device and/or a nasal sampling device. In one embodiment, a nanosorption device comprises a membrane, preferably a chemically inert, synthetic, and/or absorptive membrane, more preferably a membrane which is chemically inert, synthetic, and absorptive. In one embodiment, the nanosorption device with membrane allows maximal elution of the membrane without withhold of analytes. In one embodiment, a nasal sampling device comprises and/or consists of filter paper stripes, preferably filter paper stripes made of a synthetic membrane. In one embodiment, said filter paper stripes do not bind proteins. In one embodiment, the sample, e.g. nasal secretion, is not obtained using nasal scraping.

In one embodiment, a kit of the invention is for assessing an allergic response, such as predicting and/or monitoring therapeutic success of an allergen-specific immunotherapy (AIT) in a patient suffering from or having a disposition to develop an allergic disease such as hay fever, allergic rhinitis, allergic asthma, allergic conjunctivitis, food allergy and stinging insect hypersensitivity. In one embodiment, a kit of the invention is used for performing a method of predicting and/or monitoring therapeutic success of an allergen-specific immunotherapy of the invention. In one embodiment, said kit is used for performing a method of determining an allergic response to an allergen in a patient of the invention. In one embodiment, a method of predicting and/or monitoring therapeutic success of an allergen-specific immunotherapy of the invention and/or a method of determining an allergic response to an allergen in a patient of the invention comprise(s) using the kit of the invention. In one embodiment, the use of the invention comprises using the kit of the invention. In one embodiment, the use of the invention involves a method of predicting and/or monitoring therapeutic success of an allergen-specific immunotherapy of the invention and/or a method of determining an allergic response to an allergen in a patient of the invention.

In one embodiment, a method of the invention is performed as a point-of-care method, for example by using a kit of the invention. In one embodiment, a method of the invention comprises using a point-of-care device, such as a capillary test system, and/or a kit of the invention. The terms "a method of the invention" and "methods of the invention", as used herein, relate to a method of predicting and/or monitoring therapeutic success of an allergen-specific immunotherapy (AIT) in a patient and/or to a method of determining an allergic response to an allergen in a patient.

The term "NOG", as used herein, relates to the protein noggin and/or a gene encoding the protein noggin. In one embodiment, IL-4, IL-13 and IL-5 are produced by lymphocytes that infiltrate the epithelium. In one embodiment, a method of the invention comprises determining a seasonal response for determining an allergic response to seasonal allergens such as pollen. In one embodiment, biomarkers CSF-1, AREG, TSLP, CD40, and/or IFN-γ are determined, preferably by measuring and/or monitoring said biomarkers over a period of time comprising at least two seasons. In one embodiment, the term "in season" is a period of time in which an allergen is exposed to a patient, such as a period of time in which pollen is in the air, e.g. spring time, and/or a period of time in which a patient has exposure to an allergen such as a food allergen or animal dander. In one embodiment, the term "off season" relates a period of time without allergen exposure, such as a period of time in which there is no allergenic pollen in the air and/or a period of time in which a patient is not exposed to an allergen, such as not exposed to a food allergen or animal dander. In one embodiment, a pollen calender can be used to determine whether a time point is "in season" for a respective allergen. In one embodiment, an "off season" time point is prior to allergen exposure and/or an "in season" time point is during or after allergen exposure. In one embodiment, in a method of predicting and/or monitoring therapeutic success of an allergen-specific immunotherapy (AIT) in a patient, if said allergen is pollen, the first sample obtained at a first time point and the second sample obtained at a second time point are obtained "off season" and "in season", respectively. In one embodiment, in a method of determining an allergic response to an allergen in a patient, if said allergen is pollen, the first sample obtained at a first time point and the second sample obtained at a second time point are obtained "off season" and "in season", respectively. In one embodiment, a first sample is obtained after a last top-dose (off season), preferably 6h after a last top-dose, and a second sample is obtained after up-dosing (in season).

As used herein, the term "comprising" is to be construed as encompassing both "including" and "consisting of', both meanings being specifically intended, and hence individually disclosed embodiments in accordance with the present invention. In one embodiment, if the wording "consisting of' is used in the context of a biomarker collection, then no further biomarkers are present in the biomarker collection apart from the biomarkers following said wording. Where used herein, "and/or" is to be taken as specific disclosure of each of the two specified features or components with or without the other.

### BRIEF DESCRIPTION OF THE FIGURES

The present invention is now further described by reference to the following figures.

All methods mentioned in the figure descriptions below were carried out as described in the examples.
**Figure 1** shows biomarkers of acute epithelial suppression. The biomarkers Serpin G1 and IL-37 are a set of biomarkers for epithelial suppression or a process of convalescence. These biomarkers drop from the initial sampling time point (6h after last top dose) compared to the second sampling time point (first in season after updosing). This time point is characterized by initial inflammatory-like processes, while these biomarkers slowly increase over the course of AIT.
**Figure 2** shows biomarkers of therapy amplitude. Biomarkers SCGB1A1, IL7, CCL-27 and CSF-₃ are increased upon allergen-specific immunotherapy. These biomarkers are thus suitable to monitor a treatment success. These markers characterize an immune- and tissue-cell response in the local airway tissue, both of regulatory, homeostatic inflammatory nature. Together, these markers are indicators of the tissue condition and therefore sensors of therapy amplitude in positive and negative direction.
**Figure 3** shows epithelial seasonal-response markers. Biomarkers POSTN, CCL26, and IL-18 are an indicator of an environmental response such as to an allergen season. Important for the symptomatic burden of patients are the seasonal symptoms and in this context, biomarkers are important that reflect the seasonal response.
**Figure 4** shows a basal stem cell and non-epithelial response. The shown biomarkers of basal stem cell and non-epithelial response are two control elements that are plastic and depend on the infiltration. The activity of basal stem cells plays an important role in airway homeostasis and therefore indicators of basal stem cell activity are important for predictive biomarker concepts.

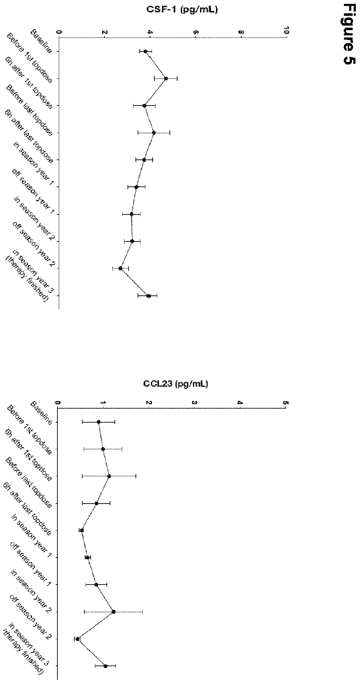

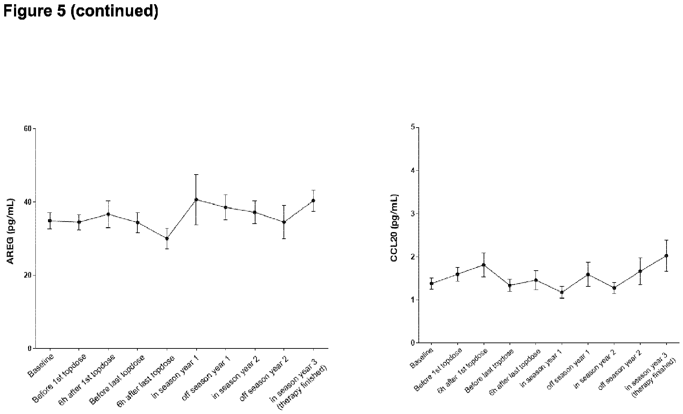

**Figure 5** shows AIT unresponsive epithelial markers. Important for any measurement are reference cytokines, which are always secreted by epithelial cells however remaining unchanged by inflammatory processes.

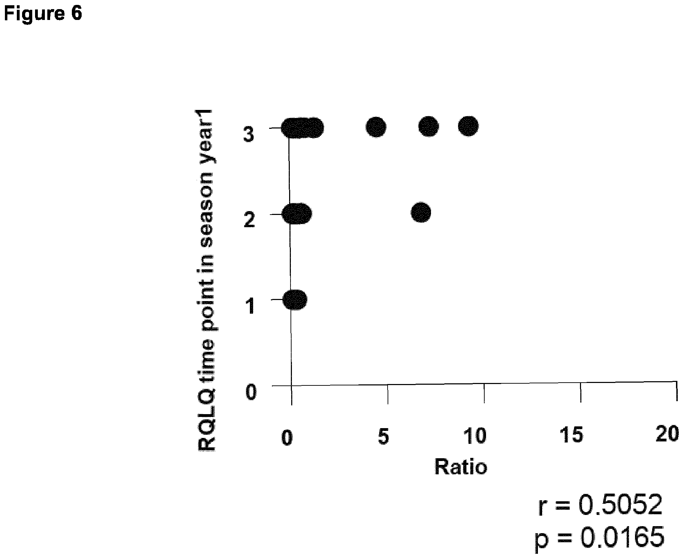

**Figure 6** shows a biomarker correlation. The biomarker levels of a set of biomarkers comprising SCGB1A1, CCL27, SerpinGi, IL37, IL24 at the time point of updosing correlates with treatment success after three years of therapy. Accordingly, the therapeutic success of an AIT can be predicted and/or monitored.

In the following, reference is made to the examples, which are given to illustrate, not to limit the present invention.

### EXAMPLES

### Example 1: Nasal secretions

Samples were obtained by using nasal absorption. The method of nasal absorption uses synthetic absorptive matrices to absorb the nasal lining fluid of the upper human respiratory tract. Nasal absorption is a non-invasive technique, which absorbs fluid from the inferior turbinate, and causes minimal discomfort. It is a form of precision mucosal sampling using a synthetic absorptive matrix for the sampling of nasal lining fluid in the upper airway. The synthetic absorptive matrices consist of polymers and is chemically inert, selected to be soft and absorptive, with rapid wicking for sample collection. Synthetic absorptive matrices have minimal protein binding to allow the efficient elution of absorbed secretions. Nasal absorption is a very gentle, non-invasive technique that can be performed on donors of all ages and allows serial sampling, even every few minutes.

### Example 2: Protein measurements

The technology used for the measurement of pro- or anti-inflammatory soluble biomarkers such as cytokines and intracellular signaling proteins in nasal secretion was based on the MSD Mesoscale platform. The markers determined by this technique allow a direct therapy monitoring. The combination of electrochemiluminescence and multi-array technology used in this platform provides unsurpassed sensitivity and multiplex functionality.

The used electrochemiluminescence detection allows the combination of sensitivity, dynamic range, and effectiveness. This detection system achieves reliable, high quality data in mucosal airway samples. High binding carbon electrodes in the bottom of microplates allow for easy attachment of biological reagents with a ten-fold higher binding capacity than polystyrene. MSD assays are based on a conventional sandwich-ELISA, however use electrochemiluminescent labels that are conjugated to detection antibodies, which allow a ultra-sensitive detection. Electricity is applied to the plate electrodes by an MSD instrument leading to light emission by the labels. Light intensity is then measured to quantify analytes in the sample. Multiple excitation cycles can amplify signals to enhance light levels, while the wide dynamic range of the detection systems means high and low expression levels can be measured without multiple sample dilutions. The stimulation method (electricity) is decoupled from the signal (light) allowing only labels near the electrode surface to be detected.

### REFERENCES

[1] Zissler UM, Jakwerth CA, Guerth F, Pechtold L, et al. (2018) Early IL-10 producing B-cells and coinciding Th/Tr17 shifts during three year grass-pollen AIT. EBioMedicine, Volume 36, P475-488.
[2] Zissler UM, Ulrich M, Jakwerth CA, et al. (2018) Biomatrix for upper and lower airway biomarkers in patients with allergic asthma. The Journal of Allergy and Clinical Immunology, Volume 142, issue 6, P1980-1983.

The features of the present invention disclosed in the specification, the claims, and/or in the accompanying figures may, both separately and in any combination thereof, be material for realizing the invention in various forms thereof.

## Claims

1. A method of predicting and/or monitoring therapeutic success of an allergen-specific immunotherapy (AIT) in a patient, wherein said method comprises the steps:
a) providing a first sample of said patient obtained at a first time point and a second sample of said patient obtained at a second time point,
b) determining, in each of said samples of said patient, a biomarker level of each biomarker of a set of biomarkers,
wherein said set of biomarkers comprises at least two, preferably at least three, more preferably at least four, even more preferably at least five biomarkers selected from a first biomarker collection consisting of SCGB1A1, IL-7, Serpin G1, IL-37, CSF-3, NOG, CCL-27, IL-24, POSTN, CCL26, and IL-18,
wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5,
c) predicting and/or monitoring therapeutic success of an allergen-specific immunotherapy in said patient, wherein a normalization of said biomarker level of one or more biomarkers of said biomarkers selected from said first biomarker collection, in said second sample compared to said first sample, indicates that said immunotherapy is successful.

2. The method according to claim 1, wherein said normalization of step c) is an approximation to and/or attainment of a reference value for the respective biomarker level, preferably a reference value of a healthy individual, and/or is an approximation to and/or attainment of a respective biomarker level determined in a reference sample, preferably a reference sample of a healthy individual.

3. A method of determining an allergic response to an allergen in a patient, wherein said method comprises the steps:
i) providing at least one sample of a patient,
wherein, optionally, said providing at least one sample comprises providing a first sample of said patient obtained at a first time point, preferably said first time point being prior to an allergen exposure, and a second sample of said patient obtained at a second time point, preferably said second time point being after said allergen exposure,
ii) determining, in said at least one sample of said patient, optionally in said first sample and said second sample, a biomarker level of each biomarker of a set of biomarkers,
wherein said set of biomarkers comprises at least two, preferably at least three, more preferably at least four, even more preferably at least five biomarkers selected from a first biomarker collection consisting of SCGB1A1, IL-7, Serpin G1, IL-37, CSF-3, NOG, CCL-27, IL-24, POSTN, CCL26, and IL-18,
wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5,
iii) determining an allergic reaction to said allergen in said patient, if said biomarker level of one or more biomarkers of said biomarkers selected from said first biomarker collection in said sample is abnormal,
wherein, optionally, said biomarker level of one or more biomarkers of said biomarkers selected from said first biomarker collection is abnormal in said second sample compared to said first sample.

4. The method according to claim 3, wherein said biomarker level being abnormal comprises said biomarker level deviating from a reference value for the respective biomarker level, preferably a reference value of a healthy individual, and/or
comprises said biomarker level deviating from a respective biomarker level determined in a reference sample, preferably a reference sample of a healthy individual, and/or
comprises said biomarker level determined in said second sample, said second sample obtained after an allergen exposure, deviating from said biomarker level determined in said first sample, said first sample obtained prior to said allergen exposure.

5. The method according to any one of the foregoing claims, wherein said set of biomarkers further comprises one or more biomarkers selected from a second biomarker collection consisting of CCL20, CCL23, CSF-1, AREG, TSLP, CD40, IFN-γ, IL-4, IL-13, and IL-5, and wherein said method comprises a step of standardizing said biomarker level of one or more biomarkers of said biomarkers selected from said first biomarker collection using a biomarker level of one or more biomarkers selected from said second biomarker collection.

6. The method according to any one of claims 1-2 and 5, wherein said set of biomarkers comprises
at least one anti-inflammatory biomarker selected from SCGB1A1, IL-7, Serpin G1, IL-37, and NOG, preferably selected from SCGB1A1, Serpin G1, and IL-37,
at least one pro-inflammatory biomarker selected from CCL-27, IL-24, POSTN, CCL26, CSF-3, and IL-18, preferably selected from CCL-27 and IL-24,
optionally, a standardizing biomarker selected from CCL20, CCL23, CSF-1, AREG, TSLP, CD40, IFN-γ, IL-4, IL-13, and IL-5,
wherein, preferably, said normalization of step c) comprises an increase in said biomarker level of one or more biomarkers of said at least one anti-inflammatory biomarker in said second sample compared to said first sample, and/or comprises a decrease in said biomarker level of one or more biomarkers of said at least one pro-inflammatory biomarker in said second sample compared to said first sample.

7. The method according to any one of claims 3-5, wherein said set of biomarkers comprises at least one anti-inflammatory biomarker selected from SCGB1A1, IL-7, Serpin G1, IL-37, and NOG, preferably selected from SCGB1A1, Serpin G1, and IL-37,
at least one pro-inflammatory biomarker selected from CCL-27, IL-24, POSTN, CCL26, CSF-3, and IL-18, preferably selected from CCL-27 and IL-24,
optionally, a standardizing biomarker selected from CCL20, CCL23, CSF-1, AREG, TSLP, CD40, IFN-γ, IL-4, IL-13, and IL-5,
wherein, preferably, said biomarker level being abnormal comprises a decrease in said biomarker level of one or more biomarkers of said at least one anti-inflammatory biomarker in said sample compared to a reference value and/or reference sample, and/or comprises an increase in said biomarker level of one or more biomarkers of said at least one pro-inflammatory biomarker in said sample compared to a reference value and/or reference sample,
wherein, optionally, said reference sample is a first sample obtained prior to an allergen exposure.

8. The method according to any one of the foregoing claims, wherein said set of biomarkers comprises biomarkers SCGB1A1, CCL-27, Serpin G1, IL-37, and IL-24.

9. The method according to any one of the foregoing claims, wherein said sample is a sample noninvasively obtained from said patient, preferably a nasal secretion sample.

10. The method according to any one of the foregoing claims, wherein said biomarker levels are determined by determining a protein level and/or a nucleic acid level, preferably a protein level, of each of said biomarkers in said sample; and/or
wherein said biomarker levels are determined using a method selected from high-performance liquid chromatography (HPLC), liquid chromatography-mass spectrometry (LC/MS), enzyme-linked immunosorbent assay (ELISA), protein immunoprecipitation, immunoelectrophoresis, Western blot, protein immunostaining, microarray, PCR such as real time PCR, next generation sequencing, transcriptome-based assays, and capillary test systems.

11. The method according to any one of claims 1-2, 5-6, and 8-10, wherein said allergen-specific immunotherapy comprises
- an initial induction phase, wherein an allergen is repeatedly administered to a patient in increasing doses of said allergen up to a maximum dose effective to induce immunologic tolerance to said allergen in said patient, and
- a maintenance phase, wherein said allergen is administered repeatedly to said patient at said maximum dose,
and wherein, in said method, said biomarker level of each biomarker of said set of biomarkers is determined in the first 1 - 7 weeks of the maintenance phase after the initial induction phase.

12. The method according to any one of claims 1-2, 5-6, and 8-11, wherein therapeutic success is measured by a patient-assessed retrospective assessment of seasonal allergic symptoms (RAAS), or by combined symptom medication score (CSMS) or by visual analog scale (VAS).

13. The method according to any one of the foregoing claims, wherein said allergen is an allergen involved in any allergic disease selected from allergic rhinitis, bee sting allergy, food allergies, hay fever, and allergic asthma, and/or wherein said allergen is selected from food allergens, pollen, in particular grass pollen, tree pollen and weed pollen, other airborne allergens, such as fungi, fungal spores, dust, mites, and animal dander.

14. A kit for predicting and/or monitoring therapeutic success of an allergen-specific immunotherapy (AIT) in a patient and/or for determining an allergic response to an allergen in a patient, comprising:
- means to obtain, preferably noninvasively obtain, a sample from said patient, wherein, optionally, said sample is a nasal secretion of said patient;
- means to perform a quantitative determination of a biomarker level of each biomarker of a set of biomarkers,
wherein said set of biomarkers comprises at least two, preferably at least three, more preferably at least four, even more preferably at least five biomarkers selected from a first biomarker collection consisting of SCGB1A1, IL-7, Serpin G1, IL-37, CSF-3, NOG, CCL-27, IL-24, POSTN, CCL26, and IL-18,
wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5,
- optionally, a set of instructions for use of said kit, said instructions indicating a therapeutic success of an allergen-specific immunotherapy (AIT) in said patient, if a biomarker level of one or more biomarkers of said biomarkers selected from said first biomarker collection is normalized in a second sample compared to a first sample; and/or
a set of instructions for use of said kit, said instructions indicating an allergic response to an allergen in said patient, if a biomarker level of one or more biomarkers of said biomarkers selected from said first biomarker collection in said sample is abnormal, preferably abnormal compared to a reference value and/or reference sample, and/or abnormal in a second sample obtained after an allergen exposure compared to a first sample obtained prior to said allergen exposure.

15. Use of a set of biomarkers for predicting and/or monitoring therapeutic success of an allergen-specific immunotherapy and/or for determining an allergic response to an allergen,
wherein said set of biomarkers comprises at least two, preferably at least three, more preferably at least four, even more preferably at least five biomarkers selected from a first biomarker collection consisting of SCGB1A1, IL-7, Serpin G1, IL-37, CSF-3, NOG, CCL-27, IL-24, POSTN, CCL26, and IL-18,
wherein, optionally, said set of biomarkers further comprises one or more biomarkers selected from a second biomarker collection consisting of CCL20, CCL23, CSF-1, AREG, TSLP, CD40, IFN-γ, IL-4, IL-13, and IL-5.
